(19)

Europäisches
Patentamt
European
Patent Office
Office européen
des brevets

(11) **EP 2 305 115 B1**

(12) **EUROPEAN PATENT SPECIFICATION**

(45) Date of publication and mention
of the grant of the patent:
**29.07.2015 Bulletin 2015/31**

(51) Int Cl.:
***A61B 5/06*** *(2006.01)*     ***A61B 18/14*** *(2006.01)*

(21) Application number: **09757088.1**

(86) International application number:
**PCT/CN2009/072122**

(22) Date of filing: **04.06.2009**

(87) International publication number:
**WO 2009/146654 (10.12.2009 Gazette 2009/50)**

(54) **METHOD FOR SIMULATING BEND SHAPE OF CATHETER AND MAGNETIC INDUCTION CATHETER**

VERFAHREN ZUR SIMULATION DER GEBOGENEN FORM EINES KATHETERS UND MAGNETINDUKTIONSKATHETER

PROCÉDÉ DE SIMULATION DE LA FORME COUDÉE D'UN CATHÉTER ET CATHÉTER À INDUCTION MAGNÉTIQUE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **06.06.2008 CN 200810038763**

(43) Date of publication of application:
**06.04.2011 Bulletin 2011/14**

(73) Proprietor: **Shanghai Microport EP Medtech Co., Ltd.**
**Shanghai 201318 (CN)**

(72) Inventors:
• **LIU, Daozhi**
**Shanghai 201203 (CN)**
• **YE, Ming**
**Shanghai 201203 (CN)**

• **GUO, Junmin**
**Shanghai 201203 (CN)**
• **WANG, Shun**
**Shanghai 201203 (CN)**

(74) Representative: **Bergmeier, Werner**
**Canzler & Bergmeier**
**Friedrich-Ebert-Straße 84**
**85055 Ingolstadt (DE)**

(56) References cited:
**WO-A1-2010/105551**     **CN-A- 1 683 025**
**CN-A- 1 802 119**     **CN-A- 1 925 787**
**CN-A- 101 347 331**     **JP-A- 2003 245 243**
**US-A1- 2007 106 114**     **US-A1- 2007 106 114**
**US-A1- 2007 106 116**     **US-A1- 2007 106 116**

**Description**

Technical Field

The present invention relates to the art of medical instruments, and more particularly to a method for simulating the bend shape of a catheter and a magnetic induction catheter.

Background of the Invention

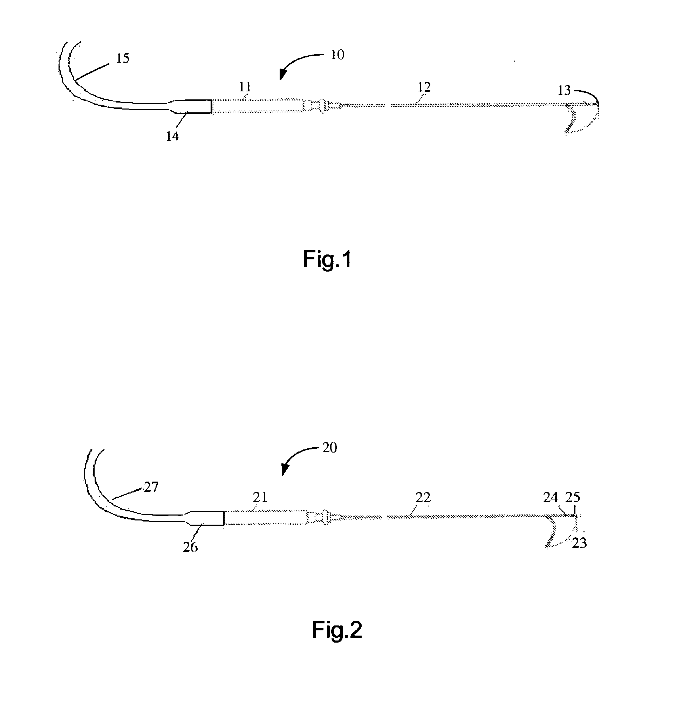

RF ablation catheters are mainly used for electrophysiological mapping, temporary cardiac pacing, RF ablating and the like of heart arrhythmia. Figure 1 is a schematic view of a known RF ablation catheter which is generally indicated by a reference numeral 10 and comprises, among others, a catheter handle 11, a catheter body 12, a tip electrode 13, a connector 14 and an extension cable 15.

In conventional catheter RF ablation treatment, by the catheter 10, the tip electrode 13 capable of transmitting RF energy may be transvenously or transarterially inserted into a human body and into a heart site under the monitoring of an X-ray TV to ablate a focus therein. When complex arrhythmia such as atrial fibrillation, atrial flutter and the like is treated, it is necessary to connect ablated points in line to completely isolate abnormal electrophysiology focus(es) for treatment purpose.

During the above linear ablation treatment, an operator needs to know the positions of the tip electrode 13 and record the positions of the focuses to be ablated to proceed with the procedure. Nowadays, three-dimensional mapping apparatus is the most often used means for mapping three-dimensional anatomical images to display the positions of the tip electrode 13 and the focuses to be ablated. Once the three-dimensional anatomical images are established, operating under X-ray is not necessary and thereby exposure to X-ray is significantly reduced. Meanwhile, in the case where the three-dimensional anatomical images are established, the ablation discharge time are reduced, thereby reducing risk of inadvertently injuring atrioventricular node, which can advantageously facilitate deployment of catheter and accurately determination of the positions of ectopic excitation and catheter. Therefore, the three-dimensional anatomical images mapped by three-dimensional mapping apparatus are more accurate and reliable than those plotted by the conventional biplane X-ray positioning.

Electric field induction and magnetic field induction are two main induction mapping methods in clinical application for mapping three-dimensional anatomical images by three-dimensional mapping apparatus. In particular, the magnetic field induction method is extensively used due to its accurate positioning and excellent repeatability. However, the three-dimensional anatomical image plotted by the present magnetic induction technology has a relatively big limitation since it can only display the position of the tip electrode 13 and can not display the bend shape of the catheter body 12 of the catheter 10 in the heart. During the procedure, the operator can not visually see the bend shape of the catheter 10 in the heart, which brings inconvenience to the procedure, thereby dramatically reducing safety and controllability thereof. US 2007/0106116 A1 discloses a device according to the preamble of claim 15.

Summary of the Invention

An object of the present invention is to provide a method for simulating the bend shape of a catheter with an electrode, which enables to display the bend shape of the catheter in use and which can bring convenience to the procedure and improve safety and controllability thereof.

Another object of the present invention is to provide a magnetic induction catheter which can display a bend shape itself in use and which can bring convenience to the procedure and improve safety and controllability thereof.

According to the present invention, a method for simulating the bend shape of a catheter which is provided with at least two sensing elements, comprises: generating an induced current by traversing magnetic lines of force with the sensing elements; extracting spatial information of the sensing elements from information of the induced current; and calculating the bend shape of the catheter based on the spatial information and in combination with characteristic information of the catheter.

Calculating the bend shape of the catheter comprises establishing a curvilinear function of the bend shape of the catheter, solving the curvilinear function of the catheter by minimal internal energy spline approximation method and obtaining the bend shape of the catheter.

Preferably, calculating the bend shape of the catheter comprises establishing a curvilinear function of the bend shape of the catheter for each catheter segment between two adjacent sensing elements, solving the curvilinear function of each catheter segment by minimal internal energy spline approximation method, obtaining the bend shape of each catheter segment, and then obtaining the whole bend shape of the catheter.

Preferably, the minimal internal energy spline approximation method further comprises regularizing the curve representing the bend shape of the catheter between two adjacent sensing elements; establishing a curvilinear function

$p(t) = at^3 + bt^2 + ct + d$ with an arc length t of the curve as a variable, in which $t$ is in a range of [0,1], and a, b, c and d are four unknowns; calculating values of $p(0), p(1)$ and $p'(0)$, $p'(1)$ based on the spatial information and characteristic information; and solving the curvilinear function $p(t)$ by substituting the values of $p(0)$, $p(1)$ and $p'(0)$, $p'(1)$ into the curvilinear function $p(t)$.

Preferably, the minimal internal energy curve method is used to solve the values of $p'(0)$, $p'(1)$

Preferably, using the minimal internal energy curve method to solve the values of $p'(0)$, $p'(1)$ comprises letting $p'(0) = a_0 v_0$, $p'(1) = a_1 v_1$, in which $a_0$, $a_1$ are lengths of tangent vectors at two ends of the curve, and $v_0$, $v_1$ are the directions of the two ends of the curve; letting $f(a_0, a_1) = E - \lambda L$, in which E represents internal energy of the curve, L represents a length of the curve, $\lambda$ is a coefficient, solving it and obtaining $a_0$, $a_1$; substituting $a_0$, $a_1$ and $v_0$, $v_1$ into $p'(0) = a_0 v_0$, $p'(1) = a_1 v_1$, and obtaining the values of $p'(0)$, $p'(1)$.

Preferably, the curve length L is a constraint condition when the curve internal energy E assumes the minimal value.

Preferably, the bend shape of the catheter is displayed.

Preferably, an inner side and an outer side of the catheter are displayed by different colors.

Preferably, a width of the catheter displayed is set based on an actual diameter of the catheter.

Preferably, one of the at least two sensing elements is arranged at a tip of the catheter.

Preferably, the spatial information comprises three-dimensional position information and direction information of the sensing elements.

Preferably, the characteristic information of the catheter comprises material, length of the catheter itself and/or an interval between two sensing elements.

Preferably, the sensing elements are magnetic sensors.

Preferably, the magnetic sensors comprise five-degree-of-freedom magnetic sensors and/or six-degree-of-freedom magnetic sensors.

According to the present invention, a magnetic induction catheter comprises a catheter body; at least two sensing elements respectively arranged on the catheter for traversing magnetic lines of force to generate an induced current; a signal extracting device for extracting spatial information of the sensing elements from information of the induced current; a simulating and processing device for calculating the bend shape of the catheter based on the spatial information and in combination with characteristic information of the catheter.

The simulating and processing device further comprises a calculating module for establishing a curvilinear function of the bend shape of the catheter, solving the curvilinear function of the catheter by minimal internal energy spline approximation method and obtaining the bend shape of the catheter.

Preferably, one of the at least two sensing elements is arranged at a tip of the catheter.

Preferably, the sensing elements are magnetic sensors.

Preferably, the magnetic sensors comprise five-degree-of-freedom magnetic sensors and/or six-degree-of-freedom magnetic sensors.

Preferably, a cable of the magnetic sensor is wrapped with a silver wire mesh.

Compared with the prior art, the present invention has the following advantages.

In the present invention, near the tip electrode of the catheter are arranged the magnetic sensors which traverse magnetic lines of force to generate an induced current. The position information and spatial information of the magnetic sensors are extracted based on the induced current information, and then the bend shape of the catheter is calculated in combination with the characteristic information of the catheter itself and displayed on the associated display device. As such, the operator can visually see the bend shape of the catheter in the heart during procedure. As compared with the prior art only displaying a position of the tip electrode of the catheter, the present invention can provide the operator with rich information of use condition of the catheter, thereby improving safety and effectiveness of the procedure.

Brief Description of the Drawings

**Fig. 1** is a schematic view of a prior-art RF ablation catheter;

**Fig. 2** is a schematic view of a catheter according to the present invention;

**Fig. 3** is a schematic sectional view of a tip electrode according to the present invention;

**Fig. 4** is an outline schematic view of the catheter according to the present invention;

**Fig. 5** is a view of the catheter in use in a heart according to the present invention;

**Fig. 6**    is a longitudinal sectional view of the catheter with three magnetic sensors mounted thereon according to the present invention;

**Fig. 7**    is a principle view of calculating bend shape of the catheter according to the present invention; and

**Fig. 8**    is a principle view of calculating the bend shape of the catheter according to the present invention.

Detailed Description of Preferred Embodiments

The above objects, features and advantages of the present invention will be apparent from the following detailed description with reference to the appended drawings and embodiments.

In the present invention, near the tip electrode of the catheter of the RF ablation catheter are arranged the magnetic sensors which traverse magnetic lines of force to generate induced current. The position information and directional information of the magnetic sensors are extracted based on the induced current information and then the bend shape of the catheter is calculated in combination with the characteristic information of the catheter itself and displayed on an associated display device. As such, the operator can visually see the bend shape of the catheter in the heart during procedure. As compared with the prior art, the present invention can bring convenience to the procedure and improve safety and controllability of the procedure.

Fig. 2 shows the schematic view of a catheter according to the present invention. The catheter 20 comprises a catheter handle 21, a catheter body 22, a tip electrode 23, a magnetic sensor 24, a magnetic sensor 25, a connector 26 and an extension cable 27. The magnetic sensors 24 and 25 are arranged within the catheter body 22 in a short distance from the tip electrode 23. Specifically, the magnetic sensor 24 is arranged at a tip of the catheter body 22. The tip electrode 23 is connected with the catheter handle 21 via the catheter body 22. The catheter handle 21 is connected with the extension cable 27 via the connector 26. The extension cable 27 is connected with a three-dimensional mapping apparatus and the like.

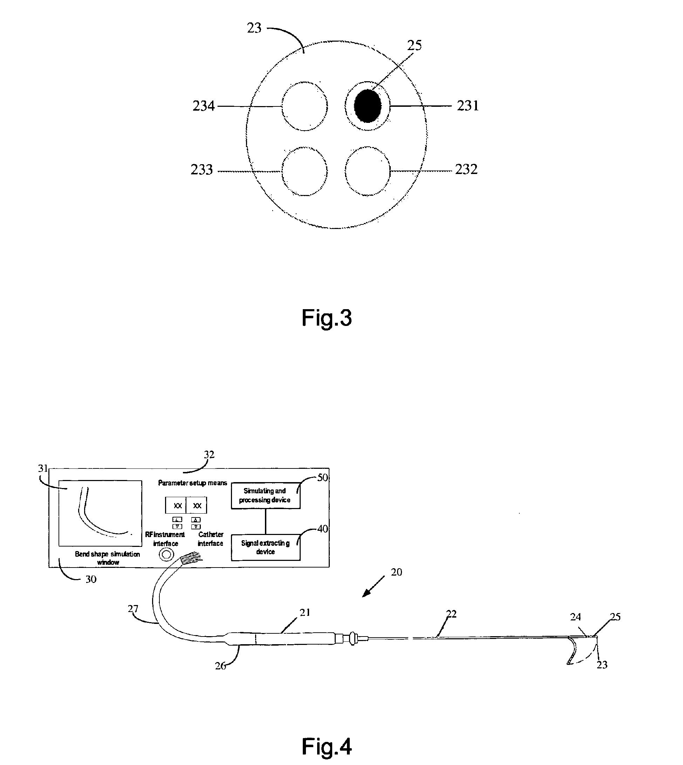

Fig. 3 shows the schematic sectional view of the tip electrode 23. The tip electrode 23 comprises a magnetic sensor lumen 231, a cold saline perfusion lumen 232, a lead wire lumen 233, and a pull wire lumen 234, wherein the magnetic sensor 25 is hermetically mounted by glue or the like in the magnetic sensor lumen 231.

The tip electrode 23 may be used for ablating, mapping, and simulating and the like. The tip electrode 23 is a cylinder having a length in a range of 3mm-8mm and made of platinum, platiniridium, stainless steel and the like. Lead wires for transmitting RF energy, temperature sensors for measuring temperature, pull wires for controlling the bend shape of the tip section of the catheter, or tubes for perfusing saline and the like may be fixed in the tip electrode 23.

The magnetic sensors 24 and 25 may be both five-degree-of-freedom magnetic sensors each composed of a single coil, or may be both six-degree-of-freedom magnetic sensors each composed of three coils perpendicular to each other, or may be respectively a five-degree-of-freedom magnetic sensor and a six-degree-of-freedom magnetic sensor. Both of the magnetic sensors 24 and 25 have connecting cables. The five-degree-of-freedom magnetic sensor has two connecting lead wires, and the six-degree-of-freedom magnetic sensor has six connecting lead wires. The connecting lead wires of each of the magnetic sensors are twisted with each other and tightly wrapped with connecting cable composed of silver material mesh.

Fig. 4 shows the outline schematic view of the catheter according to the present invention which comprises the catheter 20, a display device 30, a signal extracting device 40, a simulating and processing device 50, the catheter handle 21, the catheter body 22, the tip electrode 23, the magnetic sensor 24, the magnetic sensor 25, the connector 26 and the extension cable 27. The display device 30 comprises a bend shape simulation window 31 and a parameter setup means 32.

When the catheter 20 is used for treatment, a heart of a patient is located at an optimal working region of a magnetic field generator. As the catheter 20 is moved in the heart, the magnetic sensors 24 and 25 within the catheter body 22 are moved to traverse magnetic lines of force to generate induced current which is transmitted to a signal amplifier (not shown) in the catheter handle 21 via the connecting lead wires. The current signal is amplified by the signal amplifier and then is transmitted to the signal extracting device 40. By the signal extracting device 40, position information and direction information of the magnetic sensors 24 and 25 are extracted from the information of the induced current, and then the same are transmitted to the simulating and processing device 50.

The bend shape of the catheter 20 is calculated by the simulating and processing device 50 based on the position information and direction information in combination with the characteristic information of the catheter 20, and then is transmitted to the display device 30 via the extension cable 27. The simulating and processing device 50 further comprises a calculating module for establishing a curvilinear function of the bend shape of the catheter, solving the curvilinear function of the catheter by minimal energy spline approximation method based on the position, direction and characteristic information, and obtaining the bend shape of the catheter.

The display device 30 shows the bend shape of the catheter 20 via the flexing simulation window 31.

The diameter and color of the catheter 20 that are displayed by the bend shape simulation window 31 can be changed by adjusting the setup parameter via parameter setup means 32 of the display device 30. For example, the bend shape of the catheter 20 may be displayed realistically by setting a corresponding display width based on the actual diameter of the catheter 20. An inner side and an outer side of the bend shape of the catheter 20 may be differentiated by the colors by means of the parameter setup means 32 so as to make the display stereoscopic.

In the present invention, a distance between the magnetic sensor 25 and the tip electrode 23 is taken as a known parameter. As the catheter 20 is moved during the procedure, the position information of the tip electrode 23 is obtained based on the position information of the magnetic sensor 25 and the distance between the magnetic sensor 25 and the tip electrode 23. When the tip electrode 23 contacts with various sites of the heart chamber as the catheter 20 is moved, the profile structure of the heart chamber can be depicted, and the information of the ablated points and the cardiac electrophysiological activities are indicated in the endocardiac surface.

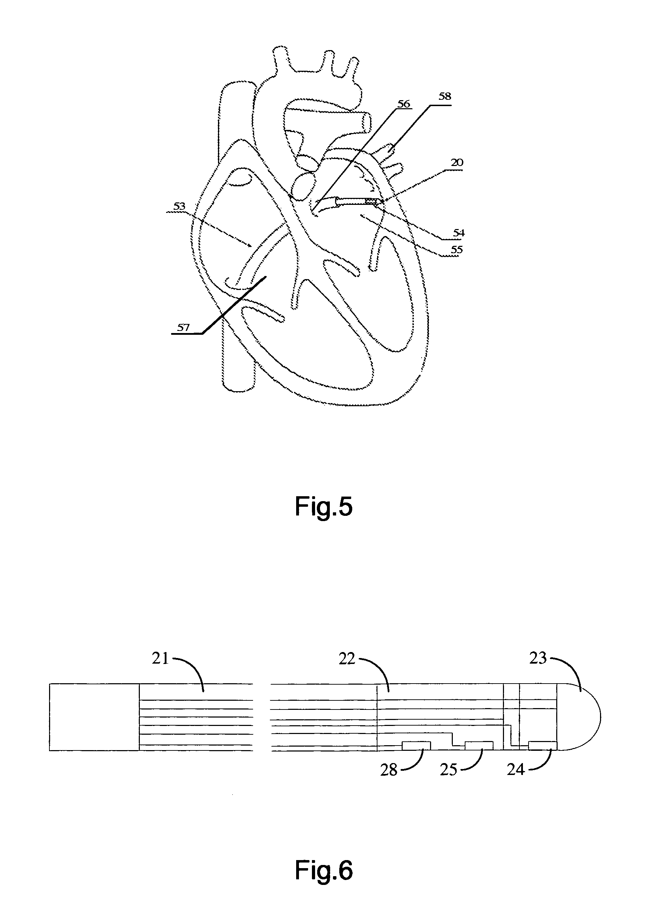

Referring to Fig. 5, which is the view of the RF ablation catheter in use in a heart according to the present invention. A sheath tube 53 penetrates through femoral vein, into a right atrium 55 from inferior vena cava 54, then penetrates through atrial septum 55 into a left atrium 57. The catheter 20, passing through the sheath tube 43, performs RF ablation near an orifice of pulmonary vein 58. The catheter 20 is operated under the guidance of the heart model and with reference to the bend shapes of the catheter 20, so that the performance of the procedure can be visually controlled.

By displaying the position of the tip electrode 23 and the bend shape of the catheter 20 to represent the bend shapes of the catheter 20 in the heart chamber, the present invention can provide the operator with rich use condition information of the catheter 20, thereby improving safety and effectiveness of the procedure.

In the present invention, more magnetic sensors may be further provided at the region in a short distance from the tip electrode 23 of the catheter 20. Preferably, the number of the magnetic sensors is 2-4 by taking into consideration the cost factor and assembly space.

Referring to Fig. 6 which is the longitudinal sectional view of the catheter with three magnetic sensors mounted thereon according to the present invention. The catheter 20 comprises the catheter handle 21, the catheter body 22, the tip electrode 23, the magnetic sensor 24, the magnetic sensor 25, and the magnetic sensor 28. The magnetic sensors 24, 25 and 28 are arranged within the catheter body 22. More specifically, the magnetic sensor 24 is arranged at the tip of the catheter body 22, and the magnetic sensors 25 and 28 are arranged in a short distance from the magnetic sensor 24.

Certainly, instead of the magnetic sensors, the present invention may also use other sensing elements having a function of magnetic field induction.

In the present invention, the simulation and calculation of the bend shapes of the catheter 20 is mainly performed by the signal extracting device 40 and the simulating and processing device 50 which can be integrated into a single chip disposed within the catheter handle 21.

The working principle of the simulating and processing device 50 will be described in detail by way of example as follows.

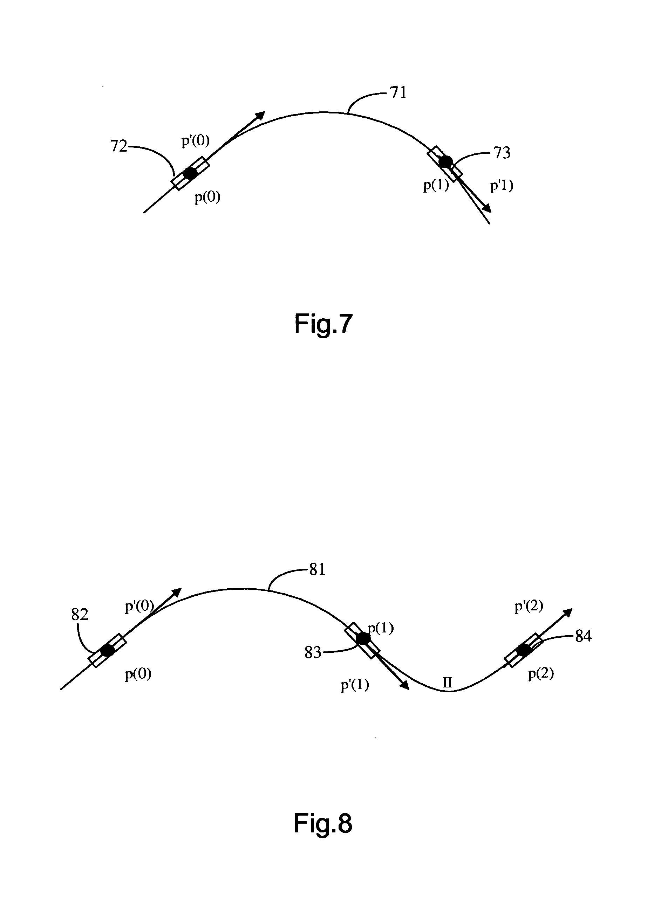

Referring to Fig. 7, which is the principle view of calculating the bend shape of the catheter according to the present invention. Provided that the trajectory of the bend shape of the catheter 20 is a simple three-dimensional curve 71, and a rectangular box (sensor) 72 and a rectangular box 73 corresponding respectively to the two magnetic sensors are provided on the curve 71.

Regularizing the curve 71 with the rectangular boxes, taking an arc length $t$ of the curve as a variable with a value in a range of [0,1], then the curvilinear function is:

$$p(t) = at^3 + bt^2 + ct + d \qquad (1)$$

in which, a, b, c and d are four unknowns, the magnitudes of $p(0)$, $p(1)$ and the directions of $p(0)$, $p'(1)$ can be obtained at the signal extracting device 40, the magnitudes of $p(0)$, $p(1)$ depend on the characteristic information of the catheter 20 itself which comprises material, length of the catheter itself and an interval between the two magnetic sensors. The present invention uses minimal internal energy curve method to solve $p'(0)$, $p'(1)$. In equation (1), the unknowns a, b, c and d may be expressed as:

$$\begin{cases} a = 2p(0) - 2p(1) + p'(0) + p'(1) \\ b = -3p(0) + 3p(1) - 2p'(0) - p'(1) \\ c = p'(0) \\ d = p(0) \end{cases} \qquad (2)$$

Equation (1) may be transformed into:

$$p(t) = (2t^3 - 3t^2 + 1)p(0) + (t^3 - 2t^2 + t)p'(0) + (-2t^3 + 3t^2)p(1) + (t^3 - t^2)p'(1) \quad (3)$$

Equation (3) may be transformed into:

$$p(t) = \begin{bmatrix} t^3 & t^2 & t & 1 \end{bmatrix} \begin{bmatrix} 2 & -2 & 1 & 1 \\ -3 & 3 & -2 & -1 \\ 0 & 0 & 1 & 0 \\ 1 & 0 & 0 & 0 \end{bmatrix} \begin{bmatrix} p(0) \\ p(1) \\ p'(0) \\ p'(1) \end{bmatrix} \qquad (4)$$

In equation (4), $p'(0) = a_0v_0$, $p'(1) = a_1v_1$, $a_0$, $a_1$ are lengths of tangent vectors at two ends of the curve, and $v_0$, $v_1$ are the tangent vectors of the two ends of the curve, wherein $v_0$, $v_1$ can be obtained from the characteristic information of the catheter 20 itself. The calculation method of the lengths $a_0, a_1$ of tangent vectors at two ends of the curve will be described as follows.

Let the curvature and the curvature radius of the curve be $k(t)$ and p(t) respectively, then the internal energy of the curve is:

$$E = \int_0^1 k^2(t)ds = \int_\alpha^\beta \frac{\rho(t)d\theta}{\rho^2(t)} = \int_\alpha^\beta \frac{d\theta}{\rho(t)} \qquad (5)$$

The length of the curve is:

$$\int_\alpha^\beta \rho(t)d\theta = L \qquad (6)$$

The equation for calculating the curvature is:

$$\frac{|p''(t)|}{|1 + p'(t)^2|^{\frac{3}{2}}} \qquad (7)$$

To simplify the calculation, the internal energy of the curve may be expressed as:

$$E = \int_0^1 p''(t)^2 dt \qquad (8)$$

The length of the curve may be taken as the constraint condition when the internal energy of the curve assumes the minimal value, and Lagrange multiplier method is used to solve (the constraint condition may not be taken into account for simplifying the calculation):

$$f(a_0, a_1) = E - \lambda L \qquad (9)$$

According to equation (4), let:

$$U = 2p_0 - 2p_1 + a_0 v_0 + a_1 v_1 \qquad (10)$$

$$V = -3p_0 + 3p_1 - 2a_0 v_0 - a_1 v_1 \qquad (11)$$

Substitute equations (10) and (11) into equation (8) to obtain:

$$E = 12U^2 + 12UV + 4V^2 \qquad (12)$$

Solve the equations:

$$\begin{cases} \dfrac{\partial f(a_0, a_1)}{\partial a_0} = 0 \\[2mm] \dfrac{\partial f(a_0, a_1)}{\partial a_1} = 0 \\[2mm] \dfrac{\partial f(a_0, a_1)}{\partial \lambda} = 0 \end{cases}$$

Then obtain the lengths of the tangent vectors at the two end of the curve:

$$a_0 = \frac{6[(p_1 - p_0) \bullet v_0](v_1)^2 - 3[(p_1 - p_0) \bullet v_1](v_0 \bullet v_1)}{[4(v_0)^2(v_1)^2 - (v_0 \bullet v_1)^2]} \; ; \; (13)$$

$$a_1 = \frac{3[(p_1 - p_0) \bullet v_0](v_0 \bullet v_1) - 6[(p_1 - p_0) \bullet v_1](v_0)^2}{[(v_0 \bullet v_1)^2 - 4(v_0)^2(v_1)^2]} \; ;$$

By obtaining the lengths $a_0, a_1$ of the tangent vectors at the two ends of the curve, the values of the $p'(0)$, $p'(1)$ can be obtained, and then the bend shape of the curve can be obtained from equation (4) in combination with the values of $p(0), p(1)$.

If the catheter is rather long or more realistic bend shape of the catheter is needed, three or more sensing elements may be provided. A curvilinear function of the bend shape of the catheter is established for each catheter segment between two adjacent sensing elements. The curvilinear function of each catheter segment is solved by minimal energy spline approximation method, as a result, the bend shape of each catheter segment and then the whole bend shape of the catheter can be obtained.

When the bend shape of the catheter is rather complex, for example, there are two or more continuous bend segments, the bend shape of the catheter may be divided into multiple segments with simple 3D curves.

Fig. 8 shows the principle view of calculating the bend shape of the catheter according to the present invention. Provided that the trajectory of the bend shape of the catheter is a simple spatial curve 81, and three rectangular boxes 82, 83 and 84, respectively corresponding to three magnetic sensors, are provided thereon.

A fixed point set $(p(k), p'(k)), k = 0,1,2,\cdots,n$ is set, in which n+1 given points are divided into groups with two adjacent points as one group. The segment between two adjacent points is regularized and solved via the above equations (1)-(13) to obtain the bend shape of each segment of the curve. Then, a cubic spline curve connecting all the segments defined by the given points is obtained. The tangent vectors and their lengths at their common point of two segments of the curve are the same. This makes sure the two segments of the curve are smooth-continuous in first order.

The method for displaying the bend shape of the catheter and a catheter according to the present invention have been described in detail as above. The principles and embodiments herein have been illustrated by way of exemplified examples and the illustration of the examples aims to facilitate understanding of the method. Those skilled in the art may modify the embodiments and the applications thereof according to the present invention. The description herein shall not be construed as limitation to the present invention.

## Claims

1. A method for simulating the bend shape of a catheter with at least two sensing elements therein, comprising the steps of:

   generating an induced current by the sensing elements traversing magnetic lines of force;
   extracting spatial information of the sensing elements from the information of the induced current; and
   calculating the bend shape of the catheter based on the spatial information and in combination with characteristic information of the catheter;
   the step of calculating the bend shape of the catheter comprises establishing a curvilinear function of the bend shape of the catheter,
   solving the curvilinear function of the catheter by minimal internal energy spline approximation method and obtaining the bend shape of the catheter.

2. The method as claimed in claim 1, wherein the step of calculating the bend shape of the catheter comprises establishing a curvilinear function of the bend shape for each catheter segment between two adjacent sensing elements, solving the curvilinear function of each catheter segment by the minimal internal energy spline approximation method, obtaining the bend shape of each catheter segment and then the whole bend shape of the catheter.

3. The method as claimed in claim 2, wherein the minimal internal energy spline approximation method further comprises:

   regularizing the curve of the bend shape of the catheter between two adjacent sensing elements;
   establishing a curvilinear function $p(t) = at^3 + bt^2 + ct + d$ with an arc length $t$ of the curve as a variable, in which $t$ is in a range of [0,1], and a, b, c and d are four unknowns;
   calculating values of $p(0), p(1)$ and $p'(0) .. p'(1)$ based on the spatial information and characteristic information; and
   solving the curvilinear function $p(t)$ by substituting the values of $p(0), p(1)$ and $p'(0), p'(1)$ into the curvilinear function $p(t)$.

4. The method as claimed in claim 3, wherein the minimal internal energy curve method is used to solve the values of $p'(0), p'(1)$.

5. The method as claimed in claim 4, wherein the step of using the minimal internal energy curve method to solve the values of $p'(0), p'(1)$ comprises:

   letting $p'(0) = a_0 v_0$, $p'(1) = a_1 v_1$, in which $a_0$, $a_1$ are lengths of tangent vectors at two ends of the curve, and $v_0$, $v_1$, are the tangent vectors of the two ends of the curve;
   letting $f(a_0, a_1) = E - \lambda L$, in which E represents internal energy of the curve, L represents a length of the curve, and $\lambda$ is a coefficient, solving it and obtaining $a_0$, $a_1$ ;
   substituting $a_0$, $a_1$ and $v_0$, $v_1$ into $p'(0) = a_0 v_0$ , $p'(1) = a_1 v_1$ and obtaining the values of $p'(0), p'(1)$ .

6. The method as claimed in claim 5, wherein the length L of the curve is a constraint condition when the internal energy E of the curve assumes the minimal value.

7. The method as claimed in claim 1, further comprising displaying the bend shape of the catheter.

8. The method as claimed in claim 7, further comprising displaying an inner side and an outer side of the catheter with different colors.

9. The method as claimed in claim 7, further comprising setting a width of the catheter to be displayed based on an actual diameter of the catheter.

10. The method as claimed in claim 1 or 2, wherein one of the at least two sensing elements is arranged at a tip of the catheter in a short distance from the tip electrode.

11. The method as claimed in claim 1 or 2, wherein the spatial information comprises three-dimensional position information and direction information of the sensing elements.

12. The method as claimed in claim 1 or 2, wherein the characteristic information of the catheter comprises material, length of the catheter itself and/or an interval between two sensing elements.

13. The method as claimed in claim 1 or 2, wherein the sensing elements are magnetic sensors.

14. The method as claimed in claim 13, wherein the magnetic sensors comprise five-degree-of-freedom magnetic sensors and/or six-degree-of-freedom magnetic sensors.

15. A magnetic induction catheter (20) comprising:

    a catheter body (22),
    at least two sensing elements (24,25) respectively arranged on the catheter for traversing magnetic lines of force to generate an induced current;
    a signal extracting device (40) for extracting spatial information of the sensing elements from the information of the induced current;
    a simulating and processing device (50) for calculating the bend shape of the catheter based on the spatial information and in combination with characteristic information of the catheter;
    the simulating and processing device further comprises a calculating module for establishing a curvilinear function of the bend shape of the
    catheter, **characterized in that** the calculating module is adapted for solving the curvilinear function of the catheter by the minimal internal energy spline approximation method and obtaining the bend shape of the catheter.

16. The catheter as claimed in claim 15, wherein one of the at least two sensing elements is arranged at a tip of the catheter in a short distance from the tip electrode.

17. The catheter as claimed in claim 15, wherein the sensing elements are magnetic sensors.

18. The catheter as claimed in claim 17, wherein the magnetic sensors comprise five-degree-of-freedom magnetic sensors and/or six-degree-of-freedom magnetic sensors.

19. The catheter as claimed in claim 17, wherein a cable of the magnetic sensor is wrapped with a silver wire mesh.

**Patentansprüche**

1. Verfahren zum Simulieren der Biegeform eines Katheters mit mindestens zwei Fühlerelementen darin, umfassend folgende Schritte:

    Erzeugen eines induzierten Stroms durch die Fühlerelemente, die Magnetkraftlinien durchqueren;
    Entnehmen räumlicher Informationen der Fühlerelemente aus den Informationen des induzierten Stroms; und
    Berechnen der Biegeform des Katheters auf Grundlage der räumlichen Informationen und in Kombination mit kennzeichnenden Informationen des Katheters;
    wobei der Schritt des Berechnens der Biegeform des Katheters umfasst: Erstellen einer gekrümmten Funktion der Biegeform des Katheters, Lösen der gekrümmten Funktion durch das Spline-Näherungsverfahren der minimalen inneren Energie und Erhalten der Biegeform des Katheters.

2. Verfahren nach Anspruch 1, wobei der Schritt des Berechnens der Biegeform des Katheters umfasst: Erstellen einer gekrümmten Funktion der Biegeform für jedes Kathetersegment zwischen zwei benachbarten Fühlerelementen, Lösen der gekrümmten Funktion jedes Kathetersegments durch das Spline-Näherungsverfahren der minimalen inneren Energie, Erhalten der Biegeform jedes Kathetersegments und sodann der gesamten Biegeform des Katheters.

3. Verfahren nach Anspruch 2, wobei das Spline-Näherungsverfahren der minimalen inneren Energie weiter umfasst:

   Normalisieren der Kurve der Biegeform des Katheters zwischen zwei benachbarten Fühlerelementen;
   Erstellen einer gekrümmten Funktion $p(t) = at^3 + bt^2 + ct + d$ mit einer Bogenlänge $t$ der Kurve als Variable, in der $t$ in einem Bereich von [0,1] liegt und a, b, c und d vier Unbekannte sind;
   Berechnen von Werten von $p(0)$, p(1) sowie $p'(0)$, p'(1) auf Grundlage der räumlichen Informationen und der kennzeichnenden Informationen; und
   Lösen der gekrümmten Funktion $p(t)$ durch Substituieren der Werte von $p(0)$, $p(1)$ und $p'(0)$, $p'(1)$ in die gekrümmte Funktion p(t).

4. Verfahren nach Anspruch 3, wobei das Kurvenverfahren der minimalen inneren Energie verwendet wird, um die Werte von $p'(0)$, $p'(1)$ zu lösen.

5. Verfahren nach Anspruch 4, wobei der Schritt des Verwendens des Kurvenverfahrens der minimalen inneren Energie zum Lösen der Werte von $p'(0)$, $p'(1)$ umfasst:

   Setzen von $p'(0) = a_0v_0$, $p'(1) = a_1v_1$, worin $a_0$, $a_1$ Längen von Tangentenvektoren an zwei Enden der Kurve sind und $v_0$, $v_1$ die Tangentenvektoren der beiden Enden der Kurve sind;
   Setzen von $f(a_0, a_1) = E - \lambda L$, worin E die innere Energie der Kurve darstellt, L eine Länge der Kurve darstellt und $\lambda$ ein Koeffizient ist, Lösen der Gleichung und Erhalten von $a_0$, $a_1$;
   Substituieren von $a_0$, $a_1$ und $v_0$, $v_1$ in $p'(0) = a_0v_0$, $p'(1) = a_1v_1$ und
   Erhalten der Werte von $p'(0)$, $p'(1)$.

6. Verfahren nach Anspruch 5, wobei die Länge L der Kurve eine Bedingung ist, wenn die innere Energie E der Kurve den minimalen Wert annimmt.

7. Verfahren nach Anspruch 1, weiter das Anzeigen der Biegeform des Katheters umfassend.

8. Verfahren nach Anspruch 7, weiter das Anzeigen einer Innenseite und einer Außenseite des Katheters mit verschiedenen Farben umfassend.

9. Verfahren nach Anspruch 7, weiter das Einstellen einer Breite des anzuzeigenden Katheters auf Grundlage eines tatsächlichen Durchmessers des Katheters umfassend.

10. Verfahren nach Anspruch 1 oder 2, wobei eins der mindestens zwei Fühlerelemente an einer Spitze des Katheters in einem kurzen Abstand von der Spitzenelektrode angeordnet ist.

11. Verfahren nach Anspruch 1 oder 2, wobei die räumlichen Informationen dreidimensionale Positionsinformationen und Richtungsinformationen der Fühlerelemente umfassen.

12. Verfahren nach Anspruch 1 oder 2, wobei die kennzeichnenden Informationen des Katheters Material, Länge des Katheters selbst und/oder eines Zwischenraums zwischen zwei Fühlerelementen umfassen.

13. Verfahren nach Anspruch 1 oder 2, wobei die Fühlerelemente magnetische Sensoren sind.

14. Verfahren nach Anspruch 13, wobei die magnetischen Sensoren magnetische Sensoren mit fünf Freiheitsgraden und/oder magnetische Sensoren mit sechs Freiheitsgraden umfassen.

15. Magnetischer Induktionskatheter (20), umfassend:

   einen Katheter-Hauptteil (22);
   mindestens zwei Fühlerelemente (24, 25), die jeweils am Katheter angeordnet sind, um magnetische Kraftlinien zu durchqueren, um einen induzierten Strom zu erzeugen;
   eine Signalentnahmevorrichtung (40) zum Entnehmen räumlicher Informationen der Fühlerelemente aus den Informationen des induzierten Stroms;
eine Simulations- und Verarbeitungsvorrichtung (50) zum Berechnen der Biegeform des Katheters auf Grundlage der räumlichen Informationen und in Kombination mit kennzeichnenden Informationen des Katheters;
wobei die Simulations- und Verarbeitungsvorrichtung weiter ein Berechnungsmodul zum Erstellen einer gekrümmten Funktion der Biegeform des Katheters umfasst, **dadurch gekennzeichnet, dass** das Berechnungsmodul zum Lösen der gekrümmten Funktion des Katheters durch das Spline-Näherungsverfahren der minimalen inneren Energie und zum Erhalten der Biegeform des Katheters geeignet ist.

16. Katheter nach Anspruch 15, wobei eins der mindestens zwei Fühlerelemente an einer Spitze des Katheters in einem kurzen Abstand von der Spitzenelektrode angeordnet ist.

17. Katheter nach Anspruch 15, wobei die Fühlerelemente magnetische Sensoren sind.

18. Katheter nach Anspruch 17, wobei die magnetischen Sensoren magnetische Sensoren mit fünf Freiheitsgraden und/oder magnetische Sensoren mit sechs Freiheitsgraden umfassen.

19. Katheter nach Anspruch 17, wobei ein Kabel des magnetischen Sensors mit einem Silberdrahtnetz umwickelt ist.

## Revendications

1. Méthode pour simuler la forme courbée d'un cathéter avec au moins deux éléments senseurs incorporés en lui, comprenant les étapes suivantes :

   générer un courant induit à l'aide des éléments senseurs, traversant des lignes de force magnétiques ;
   extraire des informations spatiales des éléments senseurs depuis les informations du courant induit ; et
   calculer la forme courbée du cathéter sur la base des informations spatiales et en combinaison avec des informations de caractéristique du cathéter ;
   l'étape consistant à calculer la forme courbée du cathéter comprend le fait d'établir une fonction curviligne de la forme courbée du cathéter, de résoudre la fonction curviligne du cathéter par une méthode d'approximation spline d'énergie minimale interne et d'obtenir la forme courbée du cathéter.

2. Méthode selon la revendication 1, dans laquelle l'étape consistant à calculer la forme courbée du cathéter comprend le fait d'établir une fonction curviligne de la forme courbée pour chaque segment de cathéter entre deux éléments senseurs adjacents, de résoudre la fonction curviligne pour chaque segment du cathéter par la méthode d'approximation spline d'énergie minimale interne, d'obtenir la forme courbée de chaque segment de cathéter puis la forme courbée entière du cathéter.

3. Méthode selon la revendication 2, dans laquelle la méthode d'approximation spline d'énergie minimale interne comprend en outre les étapes suivantes :

   régulariser la courbe de la forme courbée du cathéter entre deux éléments senseurs adjacents ;
   établir une fonction curviligne $p(t) = at^3 + bt^2 + ct + d$ avec une longueur d'arc t de la courbe en tant que variable, dans laquelle t est situé dans une plage de [0,1 et a, b, c et d sont quatre inconnues ;
   calculer les valeurs de $p(0)$, $p(1)$ et de $p'(0)$, $p'(1)$ sur la base des informations spatiales et des informations de caractéristique ;
   et résoudre la fonction curviligne $p(t)$ en substituant les valeurs de $p(0)$, $p(1)$ et de $p'(0)$, p'(1) dans la fonction curviligne p(t).

4. Méthode selon la revendication 3, dans laquelle la méthode de la courbe d'énergie interne minimale est utilisée pour résoudre les valeurs de $p'(0)$ et de $p'(1)$.

5. Méthode selon la revendication 4, dans laquelle l'étape consistant à utiliser la méthode de la courbe d'énergie interne minimale pour résoudre les valeurs de $p'(0)$ et de $p'(1)$ comprend les étapes suivantes :

   déterminer $p'(0) = a_0 v_o$ et $p'(1) = a_1 v_1$, sachant que $a_0$ et $a_1$ sont des longueurs de de vecteurs tangents à deux extrémités de la courbe, et $v_0$ et $v_1$ sont les vecteurs tangents des deux extrémités de la courbe ;
   déterminer $f(a_0, a_1) = E - \lambda L$, sachant que E représente l'énergie interne de la courbe, L représente une longueur de la courbe et $\lambda$ est un coefficient, résoudre cette formule et obtenir $a_0$ et $a_1$ ;
substituer $a_0$ et $a_1$ d'une part et $v_0$ et $v_i$ d'autre part dans $p'(0) = a_0 v_0$ et $p'(1) = a_1 v_1$, et obtenir les valeurs de $p'(0)$ et de $p'(1)$.

6. Méthode selon la revendication 5, dans laquelle la longueur L de la courbe est une condition de contrainte lorsque l'énergie interne E de la courbe adopte la valeur minimale.

7. Méthode selon la revendication 1, comprenant en outre l'affichage de la forme courbée du cathéter.

8. Méthode selon la revendication 7, comprenant en outre l'affichage d'un côté intérieur et d'un côté extérieur du cathéter en différentes couleurs.

9. Méthode selon la revendication 7, comprenant en outre la détermination d'une largeur de cathéter devant être affichée sur la base d'un diamètre actuel du cathéter.

10. Méthode selon la revendication 1 ou 2, dans laquelle l'un des au moins deux éléments senseurs est disposé à un embout du cathéter, à une courte distance de l'embout de l'électrode.

11. Méthode selon la revendication 1 ou 2, dans laquelle les informations spatiales contiennent des informations de position et des informations de direction tridimensionnelles des éléments senseurs.

12. Méthode selon la revendication 1 ou 2, dans laquelle les informations de caractéristique du cathéter contiennent la matière et la longueur du cathéter lui-même et/ou un intervalle entre deux éléments senseurs.

13. Méthode selon la revendication 1 ou 2, dans laquelle les éléments senseurs sont des capteurs magnétiques.

14. Méthode selon la revendication 13, dans laquelle les capteurs magnétiques comprennent des capteurs magnétiques à cinq degrés de liberté et/ou des capteurs magnétiques à six degrés de liberté.

15. Cathéter à induction magnétique (20) comportant :

un corps de cathéter (22) ;
au moins deux éléments senseurs (24, 25) respectivement disposés sur le cathéter pour traverser des lignes de force magnétiques en vue de générer un courant induit ;
un dispositif d'extraction de signaux (40) pour extraire des informations spatiales des éléments senseurs depuis les informations du courant induit ;
un dispositif de simulation et de traitement (50) pour calculer la forme courbée du cathéter sur la base des informations spatiales et en combinaison avec des informations de caractéristique du cathéter ;
le dispositif de simulation et de traitement comportant en outre un module de calcul pour établir une fonction curviligne de la forme courbée du cathéter, **caractérisé en ce que** le module de calcul est adapté pour résoudre la fonction curviligne du cathéter par la méthode d'approximation spline d'énergie minimale interne et obtenir la forme courbée du cathéter.

16. Cathéter selon la revendication 15, dans lequel l'un des au moins deux éléments senseurs est disposé à un embout du cathéter, à une courte distance de l'embout de l'électrode.

17. Cathéter selon la revendication 15, dans lequel les éléments senseurs sont des capteurs magnétiques.

18. Cathéter selon la revendication 17, dans lequel les capteurs magnétiques comprennent des capteurs magnétiques à cinq degrés de liberté et/ou des capteurs magnétiques à six degrés de liberté.

19. Cathéter selon la revendication 17, dans lequel un câble du capteur magnétique est enveloppé dans un treillis en fil d'argent.

Fig.1

Fig.2

**Fig.3**

**Fig.4**

Fig.5

Fig.6

**Fig.7**

**Fig.8**

**EP 2 305 115 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070106116 A1